# EUROPEAN PATENT SPECIFICATION

(11) **EP 3 146 037 B1**
(45) Date of publication and mention of the grant of the patent: **18.04.2018**
(21) Application number: 15723540.9
(22) Date of filing: 22.05.2015
(51) Int. Cl.: C12N 1/20, A61K 35/747, C12R 1/245

(54) **NEW STRAIN OF LACTOBACILLUS CASEI WITH ABILITY TO DEGRADE THE IMMUNOTOXIC PEPTIDE FROM GLUTEN**
NEUER LACTOBACILLUS CASEI-STAMM MIT FÄHIGKEIT ZUM ABBAU DES IMMUNOTOXISCHEN PEPTIDS AUS GLUTEN
NOUVELLE SOUCHE DE LACTOBACILLUS CASEI POUVANT DÉGRADER LE PEPTIDE IMMUNOTOXIQUE PROVENANT DU GLUTEN

(30) Priority: 23.05.2014 ES 201430768
(43) Date of publication of application: 29.03.2017
(73) Proprietor: Consejo Superior de Investigaciones Cientificas (CSIC), 28006 Madrid (ES)
(72) Inventor: ALVAREZ SIEIRO, Patricia, E-28006 Madrid 28 Madrid (ES); MARTÍN MARTÍN, Maria, Cruz, E-28006 Madrid (ES); REDRUELLO TRELLES, Begoña, E-28006 Madrid (ES); LADERO LOSADA, Víctor, E-28006 Madrid (ES); FERNÁNDEZ GARCÍA, María, E-28006 Madrid (ES); ÁLVAREZ GONZÁLEZ, Miguel, Ángel, E-28006 Madrid (ES)
(74) Representative: ZBM Patents ApS
(86) International application number: PCT/EP2015/061467
(87) International publication number: WO 2015/177366

(56) References cited:
- WO-A1-2011/110884
- ALBERTO CAMINERO ET AL: "Diversity of the cultivable human gut microbiome involved in gluten metabolism: isolation of microorganisms with potential interest for coeliac disease", FEMS MICROBIOLOGY ECOLOGY, vol. 88, no. 2, 3 March 2014 (2014-03-03), pages 309-319, XP055207689, ISSN: 0168-6496, DOI: 10.1111/1574-6941.12295
- M. FERNANDEZ-FEO ET AL: "The cultivable human oral gluten-degrading microbiome and its potential implications in coeliac disease and gluten sensitivity", CLINICAL MICROBIOLOGY AND INFECTION, vol. 19, no. 9, 1 September 2013 (2013-09-01), pages E386-E394, XP055207635, ISSN: 1198-743X, DOI: 10.1111/1469-0691.12249
- GEREZ C L ET AL: "Functionality of lactic acid bacteria peptidase activities in the hydrolysis of gliadin-like fragments", LETTERS IN APPLIED MICROBIOLOGY, PUBLISHED FOR THE SOCIETY FOR APPLIED BACTERIOLOGY BY BLACKWELL SCIENTIFIC PUBLICATIONS, vol. 47, no. 5, 1 November 2008 (2008-11-01), pages 427-432, XP002607421, ISSN: 0266-8254, DOI: 10.1111/J.1472-765X.2008.02448.X
- RIZZELLO CARLO G ET AL: "Highly efficient gluten degradation by lactobacilli and fungal proteases during food processing: New perspectives for celiac disease", APPLIED AND ENVIRONMENTAL MICROBIOLOGY, AMERICAN SOCIETY FOR MICROBIOLOGY, US, vol. 73, no. 14, 1 July 2007 (2007-07-01), pages 4499-4507, XP002607417, ISSN: 0099-2240, DOI: 10.1128/AEM.00260-07 [retrieved on 2007-05-18]
- ANGELIS M D ET AL: "VSL#3 probiotic preparation has the capacity to hydrolyze gliadin polypeptides responsible for Celiac Sprue probiotics and gluten intolerance", BIOCHIMICA ET BIOPHYSICA ACTA. MOLECULAR BASIS OF DISEASE, AMSTERDAM, NL, vol. 1762, no. 1, 1 January 2006 (2006-01-01), pages 80-93, XP027998079, ISSN: 0925-4439, DOI: 10.1016/J.BBADIS.2005.09.008 [retrieved on 2006-01-01]

## Description

### FIELD OF THE INVENTION

The present invention belongs to the food and pharmaceutical industry sector. The strain claimed can be applied to the degradation of gluten, gliadin and peptides thereof, during the preparation of fermented, functional foods and new foods. It can also be used as a probiotic, symbiotic, nutraceutical or dietary supplement, and in the preparation of pharmaceutical formulations with clinical applications.

### BACKGROUND ART

Celiac disease is a chronic disorder of the small intestine of autoimmune character which is induced by the ingestion of gluten in genetically predisposed individuals. Typical symptoms are associated with bowel disturbances due to the atrophy of intestinal villi and crypt hyperplasia, causing a drastic reduction in the surface of the intestinal wall (malabsorption, diarrhea, anemia, fatigue, weight loss, bloating abdominal, etc.). In most cases extraintestinal symptoms also occur (osteoporosis, depression, infertility, etc.), also gluten ingestion and celiac disease are closely associated with other disorders such as diabetes mellitus type I, Down syndrome, multiple sclerosis, dermatitis herpetiformis, myopathy, arthritis, autism, schizophrenia, lymphomas, etc. Celiac disease is widely distributed throughout the world, being one of the most common genetic diseases in which genetic and environmental factors (gluten) are involved. It is estimated a prevalence of 1% in the general population, of which only 1 in every 7-10 patients and 10 to 12% in first-degree relatives are well-diagnosed (Book et al., 2003. Prevalence of celiac disease among relatives of sib pairs with celiac disease in U.S. families. Am J. Gastroenterol. 93; 377-81; Gujral et al., 2012. Celiac disease: Prevalence, diagnosis, pathogenesis and treatment. World J Gastroenterol. 14; 18(42): 6036-6059). Although so far gluten intolerance was associated to European and American population, recent data show a significant increase in numbers of affected people in other countries such as China.

The main environmental factor triggering celiac disease and associated disorders is gluten. Gluten is composed of a set of proteins found in grains of different cereals: wheat, barley, rye and oats. Gluten proteins can be classified into two groups depending on their solubility in water-alcohol solutions: prolamins (soluble fraction) and glutenins (insoluble fraction) (Wieser 2007. Chemistry of gluten proteins. Food microbiol. 24; 115-119). Most of the peptide sequences conforming the toxic epitopes involved in triggering celiac disease are prolamins, which receive a different name depending on the cereal of origin: gliadin for wheat, hordeins in barley, secalin in rye and avenins in oats. Gluten multiproteic structure gives some technological characteristics of great importance as water absorption, gas retention, cohesiveness, elasticity and viscosity. Consequently, gluten is an essential ingredient in many food products, being present in 70% of manufactured foods, for which a suitable substitute has not yet found.

The primary structure of gliadins consists of a high percentage of proline residues (15%) and glutamine (35%), which confer high resistance to degradation by gastrointestinal enzymes. As a result, peptides resistant to the action of the gastrointestinal enzymes accumulate in the intestinal lumen (Shan et al. 2005. Identification and analysis of multivalent proteolytically resistant peptides from gluten: implications for celiac sprue. J Proteome Res. 4, 1732-1741). Among these, there is a 33 amino acid peptide (33-mer) (LQLQPFPQPQLPYPQPLPYPQPQLPYPQPQPF) which has been described as the most immunogenic peptide of gluten, by having six overlapping toxic epitopes (Qiao et al. 2004. Antigen presentation to celiac lesion-derived T cells of a 33-mer gliadin peptide naturally formed by gastrointestinal digestion. J Immunol 173:1757-1762). In susceptible individuals, these peptides are responsible for an anomalous reaction which eventually lead to chronic inflammation of the intestinal mucosa, crypt hyperplasia and a progressive deterioration of intestinal villi and even their total disappearance.

Among the genetic factors, the major histocompatibility complex (HLA) is involved. Celiac disease is associated with the expression of HLA-DQ2 in over 90% of patients and HLA-DQ8 in most other. Toxic peptides once deaminated are recognized by HLA-DQ2 and HLA-DQ8 molecules. These molecules present peptides to CD4+ T lymphocytes that will activate causing an adaptive immune response (Hausch et al. 2002. Intestinal digestive resistance of immunodominant gliadin peptides. Am J Physiol Gastroint Liver Physiol. 238:G996-1003). In addition, gluten peptides can also trigger the mechanisms of innate immunity by producing cytokine IL-15 (Maiuri et al. 2003. Association between innate response to gliadin and activation of pathogenic T cells in coeliac disease. Lancet. 362(9377):30-7).

Despite the severity of symptoms and the high prevalence of the disease, an effective therapy has not yet developed. The only treatment currently available for celiac disease is a strict gluten-free diet throughout the patient's life. Completely avoid eating gluten is a complicated task because gluten traces are found in over 80% of the food we eat. Furthermore, gluten-free products have a high cost. This increases the difficulty of following this diet especially for some sectors of the population such as children and the elderly. In order to facilitate the monitoring of gluten-free diet and as food safety, the Codex Alimentarius Comission requires that a food is labeled as "exempt or gluten-free" when it has less than 20 ppm (equivalent to 10 ppm of gliadin), and as "gluten low content" when it has less than 100 ppm. However, some celiac patients are extremely sensitive, so even eating gluten traces present in food may constitute a severe health risk.

Advances in the understanding of the molecular and cellular mechanisms of the disease has led to the development of new therapeutic options. Some of these strategies act on the immunostimulatory effects of gluten in the intestinal mucosa, for example by inhibiting tissue transglutaminase (Khosla et al. 2009. Transglutaminase inhibitors and methods of use thereof. US7579313 B2), blocking antigen presentation to HLA-DQ2 and HLA-DQ8 molecules (Sollid et al. 2007. Drug therapy for celiac sprue. US20070161572/A1) or modulating the action of cytokines such as INF or IL-15 (Sandborn y Targan. 2002. Biologic therapy of inflammatory bowel disease. Gastroenterol. 122(6):1592-608). These strategies modify molecules involved in numerous biological processes so their handling would produce adverse side effects. Alternatives aimed to modulate the abnormal immune response caused by the interaction of the toxic peptides of gluten with the immunocompetent cells of the individual are also being studied (Laparra et al., Bifidobacterium longum CECT 7347 modulates immune responses in a gliadin-induced enteropathy animal model. PLoS One. 2012;7(2):e30744).

Another alternative to fight celiac disease is to avoid the presence of toxic peptides in food, having raised several strategies:
- The selection of wheat varieties containing less content of toxic epitopes (Spaenij-Dekking et al. 2005. Natural variation in toxicity of wheat: potential for selection of nontoxic varieties for celiac disease patients. Potential for selection and breeding of non-toxic wheat varieties. Gastroenterology. 129:797-806).
- The use of microorganisms able of hydrolyzing the immunotoxic peptides in bread dough (DiCagno et al. 2004. Sourdough bread made from wheat and nontoxic flours and selected lactobacilli is tolerated in celiac sprue patients. Appl Environ Microbiol 70:1088-96).
- The hydrolysis of immunotoxic peptides during the digestive process, preventing their arrival to the small intestine. For example, by oral administration of purified enzymes of vegetable or bacterial origin able of degrading gluten peptides (Piper et al. 2004. Effect of prolyl endopeptidase on digestive-resistant gliadin peptides in vivo. J Pharmacol Exp Ther.311:213-9; Tack et al. 2013. Consumption of gluten with gluten-degrading enzyme by celiac patients: a pilot-study. World J Gastroenterol. 19(35):5837-47; Gass et al. 2007. Combination enzyme therapy for gastric digestion of dietary gluten in patients with celiac sprue. Gastroenterology. 133(2):472-80).

WO2011110881 A1 discloses culture supernatants of strains of *Lactobacillus* and *Streptococcus* which have a capacity to degrade gliadin peptides involved in coeliac disease. Particularly, the percentage of degradation of the 33-mer peptide of culture supernatants from the strains *Lactobacillus casei* P02503 A1 and P02503 A3 are respectively 72.5% and 87.5% (Table 1). This means that a 27.5% and a 12.5% is 33-mer peptide which remains intact (not degraded) in the medium, thus still being immunotoxic. Caminero et al. (FEMS Microbiology Ecology 88(2):309-319, 2014) discloses several Lactobacillus strains (Lactobacillus mucosae, amylovorus and rhamnosus) isolated from the human gut and which are able to degrade the gliadin 33-mer (see Table 1 and the right-hand column on page 315). The use of said strains for the treatment of coeliac disease is proposed.

### DESCRIPTION OF THE INVENTION

### Summary of the invention

The present invention relates to a new bacterial strain, *Lactobacillus casei* IPLA12038 with deposit number CECT 8590. The strain of *Lactobacillus casei* IPLA 12038 was deposited on 15-April-2014 in the Spanish Type Culture Collection (Colección Española de Cultivos Tipo, CECT, Edificio 3 CUE, Pare Científic Universitat de Valencia, Catedrático Agustín Escardino, 9, 46980-Paterna, Valencia, Spain), by the depositor Miguel Angel Álvarez González, IPLA-CSIC, Paseo Río Linares s/n, 33300 Villaviciosa-Asturias (Spain). The strain of *L. casei* IPLA 12038 was received the accession number CECT 8590 after the International Authority of Deposit declared the strain as viable.

The depositor IPLA-CSIC, authorized the Applicant, CONSEJO SUPERIOR DE INVESTIGACIONES CIENTÍFICAS (CSIC), to refer to the aforementioned deposited biological material in the European patent application having the representative's reference number PD10109PC00 or in any subsequent patent application derived from it or claiming priority from it, and gave his unreserved and irrevocable consent to the deposited material being made available to the public in accordance with EPC Rule 33 and with any equivalent PCT Rule and/or PCT Article as from the date of filing of the aforementioned patent application.

The strain object of the invention was isolated from an acidic dough used to make bread. It was identified by molecular sequencing of 16S rRNA, getting belonging to the species *Lactobacillus casei.* The genetic fingerprint was determined by PFGE, confirming to be different from other strains within the same species and demonstrating its exclusivity.

The main advantage of the invention is that the strain *Lactobacillus casei* CECT 8590, alone, not combined with other bacterial strains, is able to completely degrade the 33-mer peptide in 12 hours of incubation. Other strains of the prior art need to be used in combination with other strains to degrade the gluten peptides and they need more time to completely degrade them. Other strains of the priort art are not able to completely degrade the 33-mer peptide, thus resulting in a pool of 33-mer peptides which are still immunotoxic. Other strains of the prior art are able to degrade other gluten peptides (such as the 20-mer, 18-mer) but not the 33-mer peptide.

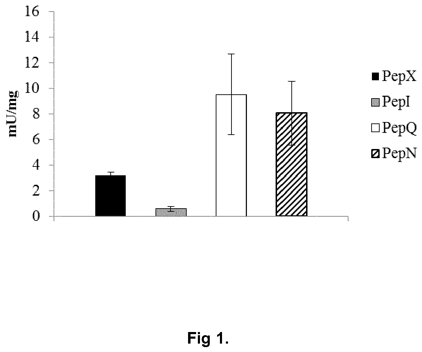

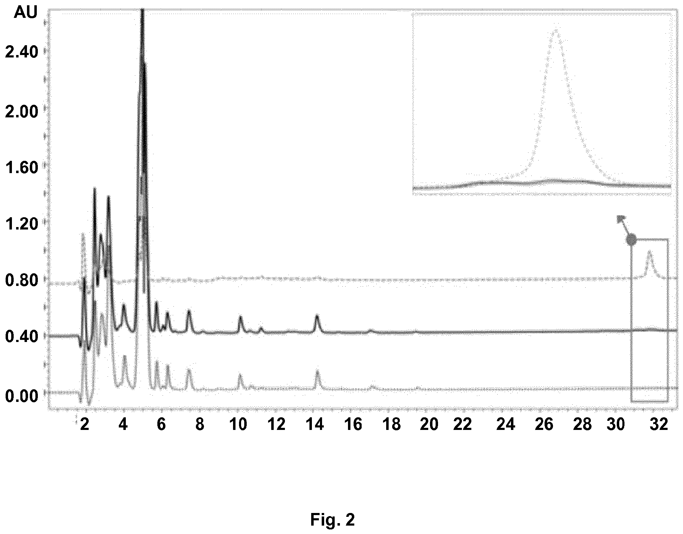

The strain of the invention possesses specific peptidase activities to hydrolyze proline residues, one of the main amino acids in gluten proteins. The combination of the different peptidases of the strain, allows total degradation of the immunotoxic 33-mer peptide (FIG. 1 and 2). Particularly, the strain has prolidase (PepQ), prolil iminopeptidase (Pepl), didpeptidil aminopetidase IV (PepX) and aminopepidase N (PepN) activities.

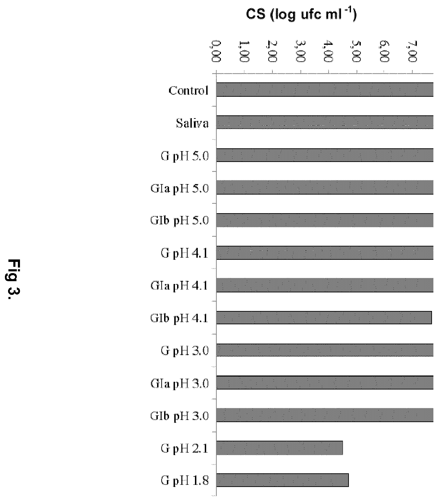

The strain object of the present invention survives the conditions of gastrointestinal stress, acid pH and high concentrations of bile salts, analyzed in vitro. These features guarantees its prolonged persistence and effectiveness in the intestine after oral administration, which facilitates its use as probiotic or food supplement (FIG. 3).

Thus, in one aspect, the invention provides a composition comprising an effective amount of the isolated strain of *Lactobacillus casei* deposited in the Spanish Type Culture Collection (CECT) under the accession number CECT 8590, wherein the strain has the ability to degrade gliadin and the immunotoxic 33-mer peptide having SEQ ID NO: 1 (LQLQPFPQPQLPYPQPLPYPQPQLPYPQPQPF), and it has been isolated from a fermented dough used in the preparation of bread.

Another aspect of the invention relates to the composition comprising the strain, as defined above, for use as a medicament or as a probiotic.

Other aspects of the invention relate to a pharmaceutical product, a veterinary product, a medical food, a food product, a food supplement or a sourdough, a probiotic food composition, a dietary supplement, a symbiotic composition, or a biotherapeutic composition, comprising an effective amount of the composition as defined above, together with appropriate amounts of acceptable excipients or food ingredients.

Finally, another aspect of the invention relates to a method to obtain a mutant of the strain of *Lactobacillus casei* deposited in the Spanish Type Culture Collection (CECT) under the accession number CECT 8590, wherein the method comprises using the deposited strain as starting material and applying mutagenesis, and wherein the obtained mutant further retains or enhances at least the ability of the deposited strain to degrade gliadin and the immunotoxic 33-mer peptide having SEQ ID NO: 1.

### Detailed description of the invention

It is an aspect of the invention a microorganism capable of degrading gliadin and the aforementioned immunotoxic peptide of 33 amino acids (33-mer). This functional property favors the hydrolysis and elimination of toxic epitopes involved in triggering of celiac disease, reducing the accumulation of immunotoxic peptides, and therefore the risk of disease development. It is characterized as a non-genetically modified microorganism, isolated from a food, non-pathogen and selected for its gluten degrading activity, hereinafter microorganism object of the present invention or strain of the invention.

The microorganism object of the present invention is also characterized by a strain belonging to the species *Lactobacillus casei* and presents additional features listed below:
- It is a microorganism generally recognized as safe (Generally Regarded As Safe, GRAS) by the FDA and QPS (Qualified Presumption of Safety) according to EFSA (Adams and Marteau. 1995. On the safety of lactic acid bacteria from food. Int J Food Microbiol. 27, 263-264).
- It is part of human normal gastrointestinal microbiota and for years has been widely used in fermented foods and as probiotics to improve the health of its host (Tuohy et al. 2003. Using probiotics and prebiotics to gut health Improve Drug Discov Today 8:692-700).
- Bacteria belonging to the genus *Lactobacillus* have been used to treat inflammatory bowel diseases (Clarke et al. 2012. Review article: probiotics for the treatment of irritable bowel syndrome - focus on lactic acid bacteria Aliment Pharmacol Ther. 35:403-413). Specifically, studies with a strain of *Lactobacillus casei* show their ability to reduce gliadin-induced enteropathy in a murine animal model (D'Arienzo et al. 2011. Immunomodulatory effects of Lactobacillus casei administration in a mouse model of gliadin-sensitive enteropathy Scand J Immunol 74:335-341).

The strain of the invention, *Lactobacillus casei* IPLA12038 with deposit number CECT 8590, has been isolated from fermented dough used to make bread, from Poland. It was identified by molecular sequencing of 16S rRNA fragment. The sequenced fragment (1,007 bases) was amplified by PCR using the primers 27F and 1492R (Lane. 1991. 16S / 23S rRNA sequencing, 115-175. In E. Stackebrandt, and M. Goodfellow (primers ed.), Nucleic acid techniques in bacterial systematics. John Wiley & Sons, Chichester, UK). By aligning the sequence obtained with those existing in the databases maximum similarity with the equivalent sequences of strains of *L. casei* was detected. The highest identity (99%) was obtained with the *Lactobacillus casei* BL23 strain deposited in GenBank (accession number CP005486.1).

The strain of the present invention is different from others of the same species. Its genetic fingerprint was determined with the technique of electrophoresis pulsed field gel (PFGE) according to the protocol described by Smith and co-Fresno (Fresno Herrero et al., 2012. Lactobacillus casei strains isolated from cheese reduce biogenic amine accumulation in an experimental model. Int. J. Food Microbiol. 157, 297-304). With this technique the restriction profile of the chromosomal DNA of the strain was obtained, cut with the enzyme Sfil, which gives a specific profile for each strain. This indicates that an exclusive strain is provided herewith. Using the software GeneTools a dendrogram with other strains of the same species isolated from similar environments was performed, using the Dice similarity index. A 62 % homology was obtained with *Lactobacillus casei* BL23 strain deposited in GenBank (accession number FM177140.1).

The new strain of the invention has peptidases with broad specificity and peptidases with specificity for substrates containing proline such as immunotoxic gliadin that due to their high content of proline are resistant to the action of gastrointestinal enzymes. In particular, the selected strain has aminopeptidase activity (EC 3.4.11.11; PepN) (8.03 mEU/mg). Regarding the specific activities for proline residues the new strain of the invention has prolidase activity (EC 3.4.13.9; PepQ) (9.5 mEU/mg), proline iminopeptidase (EC 3.4.11.9; Pepl) (0.58 mEU/mg) and prolyl dipeptidyl aminopeptidase IV (EC 3.4.14.5; PepX) (3.19 mEU/mg) (FIG. 1).

The strain object of the invention is able to use gliadin as the sole nitrogen source for growth and development.

The microorganism object of the present invention is characterized in that it degrades the main immunotoxic peptide present in gluten. The strain is able to completely degrade the 33-mer peptide (FIG. 2). Incubation of this peptide (195 ppm) in the presence of viable cells of the selected strain allows total degradation of the peptide. After eight hours of incubation, the strain object of the present invention degraded 82 % of the immunotoxic peptide and after 12 hours the peptide was completely degraded. Thus, in a particular embodiment, the strain is able to totally degrade the immunotoxic 33-mer peptide in 12 hours.

The new strain object of the present invention is capable of surviving the passage through the *in vitro* simulated gastrointestinal tract. Resistance to gastric and gastrointestinal stress were determined (Fernández de Palencia et al. 2008. Probiotic strains: survival under simulated gastrointestinal conditions, in vitro adhesion to Caco-2 cells and effect on cytokine secretion. Eur Food Res Technol 227:1475-1484). The strain object of the present invention maintained cell viability between 10⁷-10⁸ cfu ml⁻¹ and in the most extreme conditions, pH 2 and pH 1.8, the strain maintained cell viability of 10⁴ cfu ml⁻¹ (FIG 3). These results confirm the potential use as probiotic. Furthermore, the strain was resuspended in milk, a food matrix that can act as probiotic vehicle and be a source of new functional foods.

By using the deposited strain as starting material, the skilled person in the art can routinely, by conventional mutagenesis or re-isolation techniques, obtain further mutants thereof that retain or enhance the herein described relevant features and advantages of the strains forming the composition of the invention. In one particular embodiment, the mutants are obtained by using recombinant DNA technology. In another embodiment, the mutants are obtained by random mutagenesis. Thus, another aspect of the invention relates to a method to obtain a mutant of *Lactobacillus casei* CECT8590 strain, wherein the method comprises using the deposited strain as starting material and applying mutagenesis, and wherein the obtained mutant further retains or enhances at least the ability of the deposited strain to degrade gliadin and the immunotoxic 33-mer peptide having SEQ ID NO: 1.

The general use of strains is in the form of viable cells. However, it can also be extended to non-viable cells such as killed cultures or cell lysates (obtained by exposure to altered pH, sonication, radiation, temperature, pressure, or among other means of killing or lysing bacteria) or compositions containing beneficial factors produced by the strain.

In another particular embodiment, the isolated strain has been fermented in an artificial medium and submitted to a post-treatment after the fermentation, to obtain bacterial cells, and wherein the resulting bacterial cells are in a liquid medium or in a solid form. Particularly, the post-treatment is selected from the group consisting of: drying, freezing, freeze-drying, fluid bed-drying, spray-drying and refrigerating in liquid medium.

The strain of the invention is produced by cultivating (or fermenting) the bacteria in a suitable artificial medium and under suitable conditions. By the expression "artificial medium" for microorganisms is to be understood a medium containing natural substances, and optionally synthetic chemicals such as the polymer polyvinyl alcohol which can reproduce some of the functions of serums. The artificial medium is appropriately sterilized once the substances are mixed. Accordingly, among the natural substances, common suitable artificial media are nutrient broths that contain the elements including a carbon source (e.g. glucose), a nitrogen source (e.g. amino acids and proteins), water and salts needed for bacterial growth. Growth media can be liquid form or often mixed with agar or other gelling agent to obtain a solid medium. Growth media can be also considered any food matrix where bacteria can growth under appropriate conditions, such as the ingredients to make yoghurt.

The strains can be cultivated alone to form a pure culture. The strains can also be cultivated as a mixed culture with other selected microorganisms present in the original sourdough, or together with microorganisms not present in the original sourdough. Sourdough food products refer to food products made from sourdough containing at least the strains of the invention, wherein said food products are tolerated by celiac subjects. The strain of the invention can also be obtained by cultivating bacteria of different types separately and then combining them in the desired proportions. Fermentation produces a biomass free of any other microorganisms of no interest (in clear contrast with the strains as present in nature), and can be achieved using any suitable natural and/or artificial nutrients. After cultivation, and depending on the final formulation, the strains may be used as purified bacteria, or alternatively, the bacterial culture or the cell suspension may be used, either as such or after an appropriate post-treatment. In this description, the term "biomass" refers to the bacterial strains culture obtained after cultivation (or fermentation as a term synonymous to cultivation).

By the term "post-treatment" is to be understood in the context of the present invention, any processing carried out on the biomass with the aim of obtaining storable bacterial cells. The objective of the post-treatment is decreasing the metabolic activity of the cells in the biomass, and thus, slowing the rate of cellular deleterious reactions. In other words, place the bacterial cells of the invention into an artificial quiescent, non-proliferative state in which the cells undergo almost no metabolic activity, do not replicate their genetic material, do not divide and the rate of cellular deleterious reactions is almost inexistent. This artificially-induced quiescent state cannot occur in nature. The only natural ways for prokaryotic cells to reduce their metabolic activity, cease genetic replication, and stop cell division is if the cells die, or if they transform into endospores. In contrast to the natural ways to achieve a quiescent cellular state, the human-induced fermentation and post-treatment processes described herein place the strain of the invention into a non-naturally occurring state that preserves the viability of the cells in a non-endosperm form. Thus, the deposited quiescent strain of the invention differs from their naturally-occurring counterparts.

As a result of the post-treatment, the bacterial cells can be in solid or liquid form. In solid form, the stored bacterial cells can be a powder or granules. In any case, both the solid and liquid forms containing the bacterial cells are not present in the nature, hence, are not naturally-occurring, since they are the result of artificial post-treatment process(es). The post-treatment processes may in particular embodiments require the use of one or more of so called post-treatment agent. In the context of the present invention, the expression "post-treatment agent" refers to a compound used to perform the herein described post-treatment processes. Among the post-treatment agents are to be included, without limitation, dehydrating agents, bacteriostatic agents, cryoprotective agents (cryoprotectants), inert fillers (also known as lyoprotectants), carrier material (also known as core material), etc., used alone or in combination.

There are two basic non-naturally occurring approaches to decrease the metabolic activity of the bacterial cells, and thus, two approaches to carry out the post-treatment. The first one is decreasing the rate of all chemical reactions. This can be done lowering the temperature by means of refrigerating or freezing using refrigerators, mechanical freezers, and liquid nitrogen freezers. Alternatively, decreasing the rate of all chemical reactions can also be achieved by adding substances that inhibit the growth of the bacterial cells, namely a bacteriostatic agent, abbreviated Bstatic, which is a biological or chemical agent that stops bacteria from reproducing, while not harming them otherwise, among them the bacteriostatic antibiotics and preservatives. This is in contrast to bactericides, which kill bacteria. The skilled person knows the appropriate bacteriostatic to use. As example of bacteriostatic, and without limitation, bacteriostatic antibiotics which limit the growth of bacteria by interfering with bacterial protein production, DNA replication, or other aspects of bacterial cellular metabolism, in the adequate concentrations to keep only their bacteriostatic activity. A bacteriostatic agent can also be understood as any agent that is added to modify the environmental conditions such as pH, salts concentration, or the redox state.

The second approach to carry out the post-treatment is to remove water from the biomass, a process which can involve sublimation of water using a lyophilizer. Suitable techniques to remove water from the biomass are drying, freeze-drying, spray-drying or fluid bed-drying. Post-treatments that result in solid form may be drying, freezing, freeze-drying, fluid bed-drying, or spray-drying.

The post-treatment after cultivation of the strains may consist in freezing. In this case, one or more cryoprotective agents must be added. The expressions "cryoprotective agent (or additive)" and "cryoprotectant" are used indistinctively in the present invention, and they refer to chemicals that protect the cells during freezing while minimize at the same time the detrimental effects of increased solute concentration and ice crystal formation that cause a drop in the number of viable cells and consequent losses in industrial production. The most commonly used cryoprotective agents are dimethylsulfoxide (DMSO) and glycerol, either alone or in combination; although other cryoprotectants that have been used occasionally, either alone or in combination, include: polyethylene glycol, propylene glycol, glycerine, betaine (or glycine betaine), polyvinylpyrolidone, sorbitol, dextran and trehalose. Cryoprotectants classified by families are: sulphoxides, monohydric alcohols and derivatives, diols and derivatives, triols, polyalcohols, mono-,di-,tri-,poly-saccharides, amides, N-alkylamides, imides, heterocyclic compounds, amino acids and carbonic acids, proteins, peptides, polypeptides, glycoproteins, and non-ionic surfactants. Protocols for freezing bacteria and the selection of suitable cryoprotectants are of choice and knowledge of the skilled in the art. Basically, bacteria can be conveniently cultivated or harvested from agar slants or plates, or from broth culture and harvested by centrifugation. In either case, cells are generally suspended in fresh growth medium containing the cryoprotective agent(s). Bacteria can also be concentrated by centrifugation, but are often suspended in the used medium and then diluted by adding an equal volume of fresh growth medium containing the cryoprotective agent(s). Then, the mix of cells and cryoprotectant(s) is left during at least 15 minutes but not longer than 45-60 minutes to equilibrate before the cooling process. The suspension is dispensed into vials and cooled at appropriate cooling rate.

When the post-treatment after cultivation of the strains is drying, it basically consists in drying in a desiccator in vacuo over dehydrating agents such as H₂SO₄ or P₂O₅, the microorganisms suspended in culture fluid or resuspended in saline.

The post-treatment after cultivation of the strains may consist in freeze-drying, as a method for removing water. Freeze-drying involves the removal of water from frozen bacterial suspensions by sublimation under reduced pressure. Lyophilized cultures are best maintained at 4 °C or lower. Sublimation occurs when a frozen liquid goes directly to a gaseous state without entering a liquid phase. The freeze-drying process results in a stable, readily rehydrated product. This process consists of three steps: pre-freezing the product to form a frozen structure, primary drying to remove most water, and secondary drying to remove bound water. Means and methods for freeze-drying are known in the art and at the skilled person's disposal. Due to objective and expected variability of industrial processes for manufacturing and isolation of lyophilized bacterial cultures, the latter commonly contain certain amount of inert filler also known as lyoprotectant. Its role is to standardize the content of live probiotic bacteria in the product. The following inert fillers in commercially available lyophilized cultures are used: sucrose, saccharose, lactose, trehalose, glucose, maltose, maltodextrin, corn starch, inulin, and other pharmaceutically acceptable non-hygroscopic fillers. Optionally, other stabilizing or freeze-protecting agents like ascorbic acid, are also used to form a viscous paste, which is submitted to freeze-drying. In any case, the so-obtained material can be grinded to appropriate size, including to a powder.

The post-treatment after cultivation of the strains may consist in spray-drying. Spray-drying is a process whereby a liquid formulation is converted into a dry powder in a single step. The process is typically performed by first atomizing the solution into artificially fine droplets as small as possible, maximising heat transfer and the rate of water vaporisation. Droplet sizes can range from 20 to 180 µm depending on the nozzle. There are two main types of nozzles: high pressure single fluid nozzle (50 to 300 bars) and two-fluid nozzles: one fluid is the liquid to dry and the second is compressed gas (generally air at 1 to 7 bars). The droplets are then dried quickly in a large chamber by using warm gas. The resulting dry particles are collected with a cyclone.

The post-treatment after cultivation of the strains may consist in fluid bed-drying. In the fluidized bed process first of all a carrier material (also known as core material) is needed as a basis for the substrate that should be dried. The carrier material may be, without limitation, cellulose. This carrier material is held up in the air to enable the application of the substrate via a spray nozzle. Compared to spray-drying the temperatures are lower. The bacteria are sprayed onto the dispersed carrier material in form of fine droplets. The contained water deprived by the dry air flows bottom-up through the fluidized bed. In the same time the stream of air is responsible for the fluidization of particles. Afterwards a protective coating is applied in the same matter onto the carrier that is coated with bacteria. The protective coating used must prevent bacteria cellular injury by stabilizing the cell membrane constituents, e.g. by creating a porous structure in the dried product that makes rehydration easier and contains proteins that provide a protective coating for the cells. The protective coating material may be, without limitation, skim milk and calcium alginate. The structure of an encapsulated product achieved by fluidized bed coating consists of at least three parts: inside the (spherical) carrier or core material, uniform protective coating layer, on the outside, and in between the coated active ingredient of interest, namely, the bacteria. Sometimes multiple layers of different coating materials are required to obtain the desired product properties. The size of the resulting microcapsules varies between 200 µm and 2 mm.

Alternatively to having biomass preserved in solid form, biomass may be also preserved in liquid form. This may be done by adding a bacteriostatic agent as described above to stop bacteria growth to the culture medium or with an intermediate step of harvesting cells, resuspending the pellet in saline solution with a bacteriostatic agent, and optionally refrigerating it.

Sometimes, as described for instance above in the fluid bed-drying process, the probiotic preparation is subjected to an immobilization and/or coating, or encapsulation process in order to improve the shelf life and/or functionalities. Several techniques for immobilization, coating or encapsulation of bacteria are known in the art.

In a particular embodiment of the invention, the composition comprises at least an isolated strain of the first aspect of the invention, hence, including strain CECT 8590 and mutants thereof, fermented in an artificial medium and submitted to a post-treatment after the fermentation, to obtain bacterial cells, and wherein the resulting bacterial cells are in a liquid medium or in a solid form. In a particular embodiment of the present invention, the post-treatment is selected from the group consisting of: drying, freezing, freeze-drying, fluid bed-drying, spray-drying and refrigerating in liquid medium. In a particular embodiment, the post-treatment is freeze-drying.

In a particular embodiment, the strain has been genetically modified.

A further aspect of the invention is the use of the microorganism object of the present invention in the degradation of gliadin and the immunotoxic peptides responsible for triggering celiac disease and disorders associated with the intake of gluten, and preferably of the 33 amino acids immunotoxic peptide (33-mer).

According to the above described beneficial features of strain CECT 8590, another aspect of the invention relates to any of the compositions as defined above for use in the prevention and/or treatment of celiac disease and disorders associated with the intake of gluten. This aspect can be alternatively formulated as the use of any of the compositions of the invention for the manufacture of a pharmaceutical product, a veterinary product, a medicament, a medical food, a food product or a food supplement, with the proviso that they are tolerated by celiac subjects, for the prevention and/or treatment of a condition related to alterations of the urogenital microbiota. This may be also alternatively formulated as a method for the prevention and/or treatment of celiac disease and disorders associated with the intake of gluten, comprising administering to the subject in need thereof an effective amount of any of the compositions of the invention. In a particular embodiment, disorders associated with the intake of gluten are all diseases triggered by gluten; disorders associated with the intake of gluten or gluten-related disorders include celiac disease and non-celiac gluten sensitivity (NCGS). Formerly, also gluten intolerance has been used as umbrella term, and the expression gluten sensitivity has been used either as umbrella term or for NCGS. Symptoms include bloating, abdominal discomfort or pain, diarrhea, constipation, muscular disturbances, headaches, migraines, severe acne, fatigue, and bone or joint pain.

A further aspect of the invention is relates to a pharmaceutical product, a veterinary product, a medical food, a food product or a food supplement, tolerated by a celiac subject, comprising an effective amount of any of the compositions disclosed herein together with appropriate amounts of acceptable excipients.

In the context of the present invention, all the claimed products, compositions and foods are tolerated by celiacs. The expressions "tolerated by a celiac subject", "gluten free" and "suitable for celiac subjects" have the same meaning and are used indistinctively in the present invention.

A further aspect of the present inventions is the use of the microorganism object of the present invention in the manufacture of a probiotic food composition, a dietary supplement, a symbiotic composition or a biotherapeutics composition, all of which provide a beneficial effect in reducing risks and improving the health status of patients related to the ingestion of gluten.

By "probiotic food composition" is meant any combination of ingredients, combination which is constitutive of a solid or liquid food product gluten free, which in addition to its nutritional characteristics, includes specific features that help to improve health and reduce risk of disease.

By "food supplement" is understood an additional nutritional supplement intended to complement the diet.

It is meant by "biotherapeutic composition" a product composed by a microbial preparation with a beneficial effect on health or therapeutic effect.

It is understood by "symbiotic composition" the functional food containing a mixture of prebiotic and probiotic food products.

The characteristics of the microorganism of the invention also allow its use as a technological coadjuvant in the preparation of food with reduced gluten content. Technological coadjuvant means any substance not consumed as a food in itself, but intentionally used in the processing of raw materials, foods or their ingredients to fulfill a certain technological purpose during treatment or processing, and may result in its unintentional presence, but technically unavoidable in the final product of residues of the technological coadjuvant itself or its derivatives, provided that they do not present any health risk and do not have any technological effect on the final product.

A further aspect of the invention is the use of the microorganism object of the present invention as a technological coadjuvant which promotes degradation of gliadin and immunotoxic peptides that are responsible for triggering celiac disease and disorders associated with gluten intake.

Another object of the invention is a probiotic food composition, a food supplement, a symbiotic composition or a biotherapeutic composition comprising the microorganism object of the present invention. The probiotic food composition, dietary supplement, the symbiotic composition or the biotherapeutic composition may take the form of capsules, be lyophilized, in liquid form, in pills or as gels.

The resistance of the probiotic strain of the invention to conditions of extreme acidity, makes it uniquely suited to be used at acid pH, particularly in milk products.

A further object of the invention is the use of the microorganism object of the present invention in the preparation of a pharmaceutical composition useful for the treatment of celiac disease and disorders associated with the ingestion of gluten.

Another object of the invention is a pharmaceutical composition comprising the microorganism object of the present invention, hereinafter pharmaceutical composition of the invention.

Accordingly, the composition of the invention may be prepared in any suitable form which does not negatively affect the viability of the strain forming the composition of the invention. Selection of the excipients and the most appropriate methods for formulation in view of the particular purpose of the composition is within the scope of ordinary persons skilled in the art of pharmaceutical technology. The composition can be administered orally and/or rectally.

In this sense, the composition of the invention is in solid or liquid form and may be, inter alia, in the form of powders, tablets or lozenges, sucking tablets, film preparations, solutions, aerosols, granules, pills, suspensions, emulsions, capsules, enterocoated tablets and capsules, syrups, liquids, elixirs, sweets, chewing gum, suppositories, micro-enemas.

The composition according to the invention can be formulated in a form in which the strain of the invention is the only active agent. Alternatively, the composition of the invention can be formulated mixed with one or more other active agents. Alternatively, the strains of the invention either alone or formulated mixed with one or more other active agents, can also be formulated with pharmaceutically or veterinary acceptable excipients or adequate additives or ingredients in the case of a food product. In a particular embodiment of the invention, the composition additionally contains one or more further active agents. Alternatively, the additional active agent or agents are other probiotic bacteria which are not antagonistic to the strain forming the composition of the invention.

In a particular embodiment, the composition comprises more than one active agent, the active agents may be formulated in a single pharmaceutical, veterinary, food supplement or medical food forms, tolerated by celiac subjects, or in separate forms to be administered sequential or consecutively in any order, wherein the form comprising at least one of the strains of the present invention, is administered prior to, substantially simultaneously, after or in between the administration of the other active agent(s).

Additionally, the administration can be chronic or intermittent, as deemed appropriate by the supervising practitioner, particularly in view of any change in the disease state or any undesirable side effects. "Chronic" administration refers to administration of the composition in a continuous manner while "intermittent" administration refers to treatment that is done with interruption.

The effective amount of colony forming units (cfu) for the strains in the composition will be determined by the skilled in the art and will depend upon the final formulation. For instance, when administered orally without any other active agent, the total amount of the strains of the invention is present in the composition in a single doses in amount giving an effective daily dose of from 10⁷ to 10¹² cfu, according to the current legislation, preferably from 10⁹ to 10¹¹ cfu and, when administered vaginally or rectally, in an amount giving an effective daily dose of from 10³ to 10¹² cfu, preferably from 10⁵ to 10¹⁰ cfu. The term "colony forming unit" ("cfu") is defined as number of bacterial cells as revealed by microbiological counts on agar plates. Food supplements tolerated by celiac subjects usually contain probiotic strains in an amount ranging from 10⁷ and 10¹² cfu/g. In a particular embodiment, the composition of the invention is a gluten free food supplement for daily doses comprising between 10⁹-10¹¹ cfu/g.

The term "pharmaceutical product" is understood in its widely meaning in this description, including any composition that comprises an active ingredient, in this case, the strains of the invention preferably in form of composition, together with pharmaceutically acceptable excipients. The term "pharmaceutical product" is not limited to medicaments. The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio and additionally suitable for celiacs. Each carrier, excipient, etc. must also be "acceptable" in the sense of being compatible with the other ingredients of the formulation. Suitable carriers, excipients, etc. can be found in standard pharmaceutical texts.

The term "excipient" is understood in its widely meaning in this description, including any natural or synthetic substance formulated alongside the active ingredient of a pharmaceutical product, veterinary product, a medicament, food supplement and medical food, all of them tolerated by celiac subjects. The most appropriate excipient(s) to be used depend(s) of the final formulation.

Excipients are selected, without limitation, from the group comprising: fillers/diluents/bulking agents, binders, antiadherents, disintegrants, coatings, anti-caking agents, antioxidants, lubricants, sweeteners, flavors, colours, tensides and other classes of pharmaceutically and veterinary acceptable excipients suitable for celiacs.

Fillers are selected, without limitation, from the group comprising: inulin, oligofructose, pectin, modified pectins, microcrystalline cellulose, lactose, starch, maltodextrin, saccharose, glucose, fructose, mannitol, xylitol, non-crystallizing sorbitol, calcium carbonate, dicalcium phosphate, other inert inorganic and organic pharmacologically acceptable fillers, and mixtures of these substances. At dosage form of oral suspension, fillers or diluents are selected from the group comprising: vegetable oil, oleic acid, oleyl alcohol, liquid polyethylene glycol, other pharmacologically acceptable inert liquids, or mixtures of these substances.

Binders are used in solid dosage forms, e.g. to hold the ingredients in a tablet together, to ensure that tablets and granules can be formed with required mechanical strength, and to give volume to low active dose tablets. Binders in solid dosage forms like tablets are: lactose, sucrose, corn (maize) starch, modified starches, microcrystalline cellulose, modified cellulose (for example hydroxypropyl methylcellulose (HPMC) and hydroxyethylcellulose), other water soluble cellulose ethers, polyvinylpyrrolidone (PVP) also known as povidone, polyethylene glycol, sorbitol, maltitol, xylitol and dibasic calcium phosphate; other suitable pharmacologically acceptable binders, or mixtures of these substances.

Antiadherents are used to reduce the adhesion between the powder (granules) and the punch faces and thus prevent sticking to tablet punches. They are also used to help protect tablets from sticking. The most commonly used is magnesium stearate.

As disintegrants and superdisintegrants in solid dosage forms like tablets and capsules, the following substances, without limitation, are used: cross-linked polyvinylpyrrolidone, sodium starch glycolate, sodium carboxymethyl cellulose, calcium carboxymethyl cellulose, and formaldehyde-casein, other suitable pharmacologically acceptable disintegrant and superdisintegrant, or their mixtures.

Coatings in the case of solid dosage forms, such as tablets and granules for capsules filling, protect the ingredients from deterioration by moisture in the air, make large, unpleasant-tasting tablets easier to swallow and/or in the case of enteric coatings ensure intact passage through a strong acidic medium of gastric juice (pH around 1), and which allow release in duodenum or ileum (small intestine). For most coated tablets, a cellulose ether hydroxypropyl methylcellulose (HPMC) film coating is used. Occasionally, other coating materials are used, for example synthetic polymers and co-polymers like polyvinylacetate phthalate (PVAP); co-polymers of methyl acrylate-metacrylic acid; co-polymers of methyl metacrylate-metacrylic acid;, shellac, corn protein zein or other polysaccharides, waxes or wax-like substances such as beeswax, stearic acid, higher fatty alcohols like cetyl or stearyl alcohol, solid paraffin, glycerol monostearate, glycerol distearate, or their combinations. Capsules are coated with gelatin or hydroxypropyl methylcellulose.

Enteric coatings control the rate of drug release and determine where the drug will be released in the digestive tract. Materials used for enteric coatings include fatty acids, waxes, shellac, plastics, and plant fibers and their mixtures, also in combination with other above mentioned coatings.

An anticaking agent is an additive placed in powdered or granulated materials to prevent the formation of lumps (caking) and for easing packaging, transport, and consumption. As anti-caking agents in solid dosage forms like tablets, capsules, or powders, the following are used: magnesium stearate, colloidal silicon dioxide, talc, other pharmacologically acceptable anticaking agents, or their mixtures.

Lubricants are used in solid dosage forms, in particular in tablets and capsules, to prevent ingredients from clumping together and from sticking to the tablet punches or capsule filling machine, and also in hard capsules. As lubricants talc or silica, and fats, e.g. vegetable stearin, magnesium stearate or stearic acid, and mixtures thereof, are the most frequently used lubricants in tablets or hard gelatin capsules.

Sweeteners are added to make the ingredients more palatable, especially in solid dosage forms, e.g. chewable tablets, as well as in liquids dosage forms, like cough syrup. Sweeteners may be selected from artificial, natural or synthetic or semi-synthetic sweeteners; non-limiting examples of sweeteners are aspartame, acesulfame potassium, cyclamate, sucralose, saccharine, sugars or any mixture thereof,

Flavors can be used to mask unpleasant tasting active ingredients in any dosage form. Flavorings may be natural (e.g. fruit extract) or artificial. For example, to improve: (1) a bitter product, mint, cherry or anise may be used; (2) a salty product, peach or apricot or liquorice may be used; (3) a sour product, raspberry; and (4) an excessively sweet product, vanilla.

Except auxiliary substances from the class of excipients, the formulation from the present invention can contain other pharmacologically active or nutritive substances selected from the group comprising: minerals in the form of pharmaceutically and nutritive acceptable chemical form; and L-amino acids.

Regarding the preparation of the formulations of the present invention is within the scope of ordinary person skilled in the art and will depend upon the final dosage formulation. For instance, and without limitation, when the final dosage forms is an oral solid one, such as tablets, capsules, powder, granules, oral suspension, etc. the process for preparation of solid dosage forms of the formulation includes homogenization of: (1) the active ingredient(s), comprising at least one or more post-treated probiotic bacteria of the invention in an effective amount; (2) with one or more excipients to form homogeneous mixture which is, e.g. according to requirements, subjected to lubrication with magnesium stearate or other lubricants yielding final dosage form of powder. Such homogeneous powder is filled into ordinary gelatin capsules or, alternatively, into gastro-resistant capsules. In the case of tablets, they are manufactured by direct compression or granulation. In the first case, a homogeneous mixture of active ingredients and suitable excipients such as anhydrous lactose, non-crystallizing sorbitol, and others is prepared. In the second case, tablets are processed on the mixture in granulated form. Granules are prepared by granulation process of active ingredients of the formulation with suitable fillers, binders, disintegrants, and small amount of purified water. Such prepared granules are sieved and dried until the water content of <1 % w/w.

Regarding the process for preparation of liquid dosage forms (e.g. oral suspension), it involves homogenization of the active ingredient(s) of the formulation comprising at least one or more post-treated probiotic bacteria of the invention in an effective amount in an inert liquid diluent (filler) such as various vegetable oils like sunflower, soybean or olive oil; oleic acid; oleyl alcohol; liquid polyethylene glycols like PEG 200, PEG 400 or PEG 600; or other inert pharmacologically acceptable liquids. The process further involves treatment of homogeneous mixture with one or more processes selected from the group comprising: (1) stabilization of the formulation, by addition and homogenization of suspension stabilizers like beeswax, colloidal silicon dioxide, etc.; (2) sweetening of the formulation; by addition and homogenization of sweetener; (3) flavoring of the formulation, by addition and homogenization of flavoring. Such forms of the formulation can contain also other excipients or ingredients, usually employed in the art.

The pharmaceutical product can adopt different forms or names depending on the product approval route and also depending on the country. For instance, a medicament is a particular pharmaceutical product. A medical food is another particular pharmaceutical product, wherein the medical food is gluten free. The terms "medical food" or "food for special medical purposes" are used in some countries to refer to a food specially formulated and intended for the dietary management of a disease that has distinctive nutritional needs that cannot be met by normal diet alone. They are defined in regulations such as the Food and Drug Administration's 1988 Orphan Drug Act Amendments in the United States, and the Commission Directive 1999/21/EC in Europe. Medical foods are distinct from the broader category of food supplements and from traditional foods that bear a health claim. Thus, in a particular embodiment, the composition of the invention is a medical food.

Often, probiotic bacterial compositions such as the one disclosed herein, are considered as food supplements suitable for celiacs. A food supplement suitable for celiacs, also known as dietary supplement or nutritional supplement is considered another particular pharmaceutical product. This is a preparation or product intended to supplement the diet, made from compounds usually used in foodstuffs, which provide nutrients or beneficial ingredients that are not usually ingested in the normal diet or may not be consumed in sufficient quantities. Mostly, such food supplements are considered as food products, but sometimes they are defined as drugs, natural health products, or nutraceutical products. In the sense of the present invention, food supplements also include nutraceuticals. Food supplements are usually sold "over the counter", i.e. without prescription. If the food supplement adopts the form of a pill, a capsule a tablet or a powder, it comprises excipients which are the same as the used in medicaments. A food supplement however, can also adopt the form of a food product which is fortified with some nutrients (e.g. a bar or yoghurt). Thus, in a particular embodiment, the composition of the invention is a food supplement, which must be gluten free.

If the composition according to the invention is a food supplement suitable for celiacs, it can be administered as such, can be mixed with a suitable drinkable gluten free liquid, such as water, yoghurt, milk or fruit juice, or can be mixed with solid or liquid food. In this context the food supplement can be in the form of tablets or lozenges, pills, capsules, granules, powders, suspensions, sachets, sweets, bars, syrups and corresponding administration forms, usually in the form of a unit dose. Preferably, the gluten free food supplement comprising the composition of the invention is administered in the form of tablets, lozenges, capsules or powders, manufactured in conventional processes of preparing dietary supplements.

The strains of the invention can be also included in a variety of gluten-free food products, such as a milk products (a yogurt, a cheese, a fermented milk, a milk powder, a milk based fermented product, an ice-cream, a fermented cereal based product, a milk based powder), bread, bars, spreads, biscuits and cereals, a beverage, different types of oil, or a dressing. The term "food product" is used herein in its broadest meaning, including any type of product, in any form of presentation, which can be ingested by an animal, but excluding pharmaceutical and veterinary products. Examples of other food products are meat products (e.g. sausages or meat spreads), chocolate spreads, fillings and frostings, chocolate, confectionery, baked goods (cakes, pastries), sauces and soups, fruit juices and coffee whiteners. Particularly interesting food products are food supplements and infant formulas, with the proviso that they are tolerated by celiac subjects. The gluten free food product preferably comprises a carrier material such as oat meal gruel, lactic acid fermented foods, resistant starch, dietary fibres, carbohydrates, proteins and glycosylated proteins. In a particular embodiment the strains of the invention are encapsulated or coated. Hence, in a particular embodiment, the composition of the invention is a gluten free food product. In particular, the strains of the invention can also be in the form of sourdough which is a dough containing a *Lactobacillus* culture including *L. casei* CECT 8590 in symbiotic combination with yeasts. Thus, a particular embodiment of the invention is a medium or a product fermented by the composition comprising the strain.

The pharmaceutical composition of the invention is preferably administered orally, in solid or liquid form. Illustrative examples of pharmaceutical forms for oral administration include tablets, capsules, granules, solutions, suspensions, etc., and may contain conventional excipients such as binders, diluents, disintegrants, lubricants, humectants, etc., and may be prepared by conventional methods. In any case, the excipients are chosen according to the pharmaceutical form of administration selected. A review of the different pharmaceutical forms for drug administration and their preparation can be found in the book "Tratado de Farmacia Galenica" de C. Faulí i Trillo, 10 Edition, 1993, Luzán 5, S.A. Editions, or in any book of similar characteristics that exists in each country.

In the scope of the present invention are also included those methods of treatment of celiac disease and disorders associated with the intake of gluten, which consist of the administration microorganism object of the present invention or the pharmaceutical composition of the invention, both alone or in combination with other drugs or medical techniques.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### DESCRIPTION OF THE FIGURES

**FIG. 1****.-** PepX, Pepl, PepQ and PepN enzymatic activities of *L. casei* CECT 8590 strain.
**FIG. 2****.-** Chromatogram obtained when the 33-mer peptide was incubated with *L. casei* CECT 8590 for 0 h (top line) or 12 h (middle line). Note that the peptide disappeared completely after 12 h of incubation with the strain. A reaction containing the strain alone was used to check the appearance of peptides derived from its normal metabolism (bottom line). The chromatogram section that contains the peak corresponding to the 33-mer peptide is shown enlarged in the inset.
**FIG. 3****.-** Survival to in vitro simulated gastrointestinal conditions of *L. casei* CECT 8590 strain. "CS" means cell survival. *L. casei* strain CECT 8590 was resuspended in milk and subjected to in vitro gastrointestinal conditions to determine cell survival. To simulate the gastric stress (G) was incubated with pepsin and lysozyme decreasing pH values. To simulate the gastrointestinal stress (GI) samples from the pH 5, 4.1 and 3 were incubated with bile salts and pancreatic enzymes for 20 (GIa) and 120 (GIb) minutes.

### EXAMPLES

The following examples are used to illustrate the invention and they should not be considered as limitative of the scope of the invention.

### Example 1. Isolation and selection of the strain object of the present invention

The strain of the invention, *Lactobacillus casei* CECT 8590, has been isolated from a sourdough used to make bread, from Poland. Ten grams of sample were collected and homogenized for 5 min in 90 ml Ringer saline solution (Merck, Darmstadt, Germany) using a Lab-Blender 400 stomacher (Seward Ltd., London, UK). Once homogenized, serial dilutions in Ringer saline solution were performed and 50 µl were plated in a chemically defined medium containing gliadin as sole nitrogen source (AHG-M), and incubated in (PARALLEL in) three different conditions: aerobically at 30 °C, aerobically at 37 and in anaerobiosis at 37 °C, (IN A MAC 500 ANAEROBIC WORKSTATION (DON WHITLEY SCIENTIFIC, WEST YORKSHIRE, UK)). The culture medium was supplemented with cycloheximide (50 µg ml⁻¹) to inhibit the growth of fungi and yeasts. Only 27 strains were able to grow on this medium. Of these, only 8 were able to form a halo around the colony indicating degradation of gliadin in the AHG-M medium, and according to its characteristics and to the profile determined by PFGE one was the strain object of the present invention, *Lactobacillus casei* CECT 8590.

### Example 2. Peptidase activities with synthetic substrates

The ability of the strain to hydrolyze proteins and peptides derived from gluten was carried out by quantifying the activity of intracellular peptidases of broad spectrum and with specificity to hydrolyze peptidic sequences containing proline, present in the peptides responsible for immune and toxic responses of gluten. For the preparation of cell extracts, 20 ml of bacterial culture grown 20-22 hours in MRS was used. They were collected by centrifugation (8000 g for 10 min), washed twice and resuspended in 2 ml of the buffer corresponding to the enzymatic activity to be tested. Then, the samples were broken using a French Press to 2.3 kba (Constant Cell Disruption Systems [Low March, Daventry, Northents, UK]). Cell debris were removed by centrifugation at 10,000 g at 4 for 30 min and the cell-free extracts were assayed immediately.

Peptidase activities were characterized using specific chromogenic substrates (BACHEM, BUBENDORF, SWITZERLAND). PepN, Pepl and PepX and were determined using Leu-*p-*nitroanilide (Leu-*p*-NA), Pro-*p*-NA and Gly-Pro-*p*-NA respectively. The reaction mixture contained 825 µl of 10 mM phosphate buffer (pH 7.5), 75 µl of THE ADEQUATE substrate (1.2 mM) and 150 µl of the cell extract. After 1 hour of incubation at 37 °C, the reaction was stopped by adding 500 µl of 20 % trichloroacetic acid. The released p-NA was measured spectrophotometrically at 410 nm (U-2800 DIGILAB, HITACHI HIGH-TECHNOLOGIES CORPORATION, TOKYO, JAPAN). PepQ was determined FOLLOWING THE NINHYDRIN METHOD (YARON AND MLYNAR. 1968) using Val-Pro AS A SUBSTRATE. The reaction mixture contained 100 µl of the cell extract in 162.5 µl of citrate buffer (sodium citrate [20 mM] and zinc sulfate [2.5mM]) pH 6.5, and 37.5 µl of substrate (1.2 mM). It was incubated for 30 min at 40 °C. To stop the reaction 350 µl of glacial acetic acid was added and to form the color 350 µl of the reaction mixture [ninhydrin (3 % w/v), phosphoric acid (60 % v/v) and glacial acetic acid (40 % v/v)] were added. It was incubated for 10 min at 90 °C. The color is measured spectrophotometrically at 515 nm. Enzyme assays were performed in triplicate and expressed as milliunits per mg protein, defining one unit (U) as the amount of enzyme required to release 1 µmol of p-NA, or 1 pmol of amino acid in the case of PepQ, per minute under the conditions tested. Total protein in each sample was determined using the Pierce BCA "Protein Assay" kit (PIERCE, ROCKFORD, USA). The results are presented in FIG.1.

### Example 3.- Degradation of the immunotoxic peptide of 33 amino acids

The ability of the strain to degrade the immunotoxic peptide of 33 amino acids (33-mer) responsible for the triggering of celiac disease (Shan et al. 2002 Structural basis for gluten intolerance in celiac sprue Science 297:2275-2279) was quantified by REVERSE-PHASE HIGH PERFORMANCE LIQUID chromatographic analysis (RP-HPLC). The 33-mer peptide was chemically synthesized with a purity of 95 % (Immunosteps S.L., Salamanca, Spain) and dissolved in PBS BUFFER, pH 7.4 (10 mM). Cell culture was grown in MRS BROTH at 37 °C to stationary phase. Cells were collected by centrifugation (8000 g for 10 min), they were washed twice with PBS (pH 7.4) and were resuspended in 1 ml of the same buffer. The cell suspension (OD₆₀₀ = 35) was incubated with the peptide (50 µM=195 ppm) at 37 °C and samples (200 µl) were taken to inject on HPLC (50 µl) at 0, 8 and 12 hours. The samples were heat inactivated and STORED at -20 °C until analysis. Prior to injection the samples were filtered using 0.2 µM filter membrane HT Tuffryn®. The substrate concentration as well as intermediate products were analyzed by RP-HPLC (ACCORDING TO SHAN ET AL 2002). The HPLC system consists of an Alliance 2795 separation module, PDA with a photodiode detector 2996, and software data acquisition Empower (Waters, Milford, MA). One XTerra MS C18 5 µm column, 4.6 x 150 mm column with pre column Nova-Pak C18 4 µm, 3.9 x 20 mm (Waters), with a thermostat at 30 °C was used. Elution phases consisted of (A) water with 0.1 % (v/v) TFA (trifluoroacetic acid) and (B) acetonitrile with 0.1 % (v/v) TFA. The gradient program started with 100 % of solvent A and 0 % of solvent B, and changed linearly to reach 62.6 % of solvent A and 37.4 % of solvent B in 34 minutes The column was washed with 100 % solvent B for 5 minutes and equilibrated to initial conditions for 10 min. UV absorbance at 215 nm and 280 nm was collected. The results obtained are shown in FIG. 2. *L. casei* CECT 8590 degraded the peptide by up to 82% within 8 h, and completely degraded it within 12 h (Fig. 2). Interestingly, no intermediate hydrolysis products were observed when this latter chromatogram was compared to that of a parallel incubation containing solely the strain (i.e., with no substrate).

### Example 4. Evaluation of the resistance to conditions of gastrointestinal stress

The resistance of the strain object of the invention to the acidic conditions of the gastric juices, which constitute the first barrier limiting the viability of the microorganisms following ingestion, and the action of bile salts and pancreatic enzymes was evaluated. Cell suspensions of CECT 8590 strain (108 cfu ml⁻¹) in milk were prepared. Cell cultures were grown in 30 ml MRS. The cells were recovered by centrifugation (15 min, 3000 g), washed twice with 0.85 % NaCl (w/v) and resuspended in a volume of skimmed milk powder 10 % (Oxoid). Then these cell suspensions were subjected to the *in vitro* simulated gastrointestinal tract conditions (GIT). To start with 0.1 % (w/v) lysozyme mimicking human saliva was added. Gastric stress (G) was simulated by adding pepsin (Sigma) 0.3 % (w/v) and decreasing the pH every 20 minutes (pH 5, pH 4.1, pH 3, pH 2.1 and pH 1.8). The gastrointestinal stress (GI) was tested after stress G in the samples from the pH 5, 4.1 and 3. To simulate small bowel digestion, bile salts (0.3 % w/v), a cocktail of pancreatic enzymes (1 mg ml⁻¹ of amylase, 1 mg ml⁻¹ trypsin and 1 mg ml⁻¹ of chymotrypsin) to a final concentration of 5 mg ml⁻¹ were added and the pH was raised to 6.5. Samples were taken after 20 and 120 minutes of incubation in the GI conditions. A sample was collected at each condition and serial dilutions in saline solution of 0.85 % NaCl (w/v) were performed. Samples were plated on MRS with 2 % agar and viable count was performed after incubation for 48 h at 37 °C. The results obtained are presented in FIG. 3.

### BIBLIOGRAPHIC REFERENCES

Book et al., 2003. Prevalence of celiac disease among relatives of sib pairs with celiac disease in U.S. families. Am J. Gastroenterol. 93; 377-81;
Gujral et al., 2012. Celiac disease: Prevalence, diagnosis, pathogenesis and treatment. World J Gastroenterol. 14; 18(42): 6036-6059.
Adams and Marteau. 1995. On the safety of lactic acid bacteria from food. Int J Food Microbiol. 27, 263-264.
Wieser 2007. Chemistry of gluten proteins. Food microbiol. 24; 115-119.
Shan et al. 2005. Identification and analysis of multivalent proteolytically resistant peptides from gluten: implications for celiac sprue. J Proteome Res. 4, 1732-1741.
Qiao et al. 2004. Antigen presentation to celiac lesion-derived T cells of a 33-mer gliadin peptide naturally formed by gastrointestinal digestion. J Immunol 173:1757-1762.
Hausch et al. 2002. Intestinal digestive resistance of immunodominant gliadin peptides. Am J Physiol Gastroint Liver Physiol. 238:G996-1003.
Maiuri et al. 2003. Association between innate response to gliadin and activation of pathogenic T cells in coeliac disease. Lancet. 362(9377):30-7.
Sandborn y Targan. 2002. Biologic therapy of inflammatory bowel disease. Gastroenterol. 122(6):1592-608.
Khosla et al. 2009. Transglutaminase inhibitors and methods of use thereof. US 7579313 B2.
Sollid et al. 2007. Drug therapy for celiac sprue. US 20070161572 A1.
Laparra et al., Bifidobacterium longum CECT 7347 modulates immune responses in a gliadin-induced enteropathy animal model. PLoS One. 2012;7(2):e30744.
Spaenij-Dekking et al. 2005. Natural variation in toxicity of wheat: potential for selection of nontoxic varieties for celiac disease patients. Potential for selection and breeding of non-toxic wheat varieties. Gastroenterology. 129:797-806.
DiCagno et al. 2004. Sourdough bread made from wheat and nontoxic flours and selected lactobacilli is tolerated in celiac sprue patients. Appl Environ Microbiol 70:1088-96.
Piper et al. 2004. Effect of prolyl endopeptidase on digestive-resistant gliadin peptides in vivo. J Pharmacol Exp Ther.311:213-9;
Tack et al. 2013. Consumption of gluten with gluten-degrading enzyme by celiac patients: a pilot-study. World J Gastroenterol. 19(35):5837-47.
Gass et al. 2007. Combination enzyme therapy for gastric digestion of dietary gluten in patients with celiac sprue. Gastroenterology. 133(2):472-80).
Tuohy et al. 2003. Using probiotics and prebiotics to gut health Improve Drug Discov Today 8:692-700.
Clarke et al. 2012. Review article: probiotics for the treatment of irritable bowel syndrome - focus on lactic acid bacteria Aliment Pharmacol Ther. 35:403-413.
D'Arienzo et al. 2011. Immunomodulatory effects of Lactobacillus casei administration in a mouse model of gliadin-sensitive enteropathy Scand J Immunol 74:335-341.
Lane. 1991. 16S / 23S rRNA sequencing, 115-175. In E. Stackebrandt, and M. Goodfellow (primers ed.), Nucleic acid techniques in bacterial systematics. John Wiley & Sons, Chichester, UK.
Fresno Herrero et al., 2012. Lactobacillus casei strains isolated from cheese reduce biogenic amine accumulation in an experimental model. Int. J. Food Microbiol. 157, 297-304.
Fernández de Palencia et al. 2008. Probiotic strains: survival under simulated gastrointestinal conditions, in vitro adhesion to Caco-2 cells and effect on cytokine secretion. Eur Food Res Technol 227:1475-1484.
Tratado de Farmacia Galenica" de C. Faulí i Trillo, 10 Edition, 1993, Luzán 5, S.A. Editions,
Shan et al. 2002 Structural basis for gluten intolerance in celiac sprue Science 297:2275-2279.

## Claims

1. A composition comprising an effective amount of the isolated strain of *Lactobacillus casei* deposited in the Spanish Type Culture Collection (CECT) under the accession number CECT 8590

2. The composition according to claim 1, wherein the isolated strain has been fermented in an artificial medium and submitted to a post-treatment after the fermentation, to obtain bacterial cells, and wherein the resulting bacterial cells are in a liquid medium or in a solid form.

3. The composition according to claim 2, wherein the post-treatment is selected from the group consisting of: drying, freezing, freeze-drying, fluid bed-drying, spray-drying and refrigerating in liquid medium.

4. The composition according to any of the claims 1-3, wherein the strain has been genetically modified.

5. The composition as defined in any of the claims 1-4, for use as a medicament or as a probiotic.

6. A composition as defined in any of the claims 1-4, for use in the prevention and/or treatment of celiac disease and disorders associated with gluten intake.

7. A composition as defined in any of the claims 1-4, for use as technologic coadjuvant.

8. A pharmaceutical product, a veterinary product, a medical food, a food product, a food supplement or a sourdough, a probiotic food composition, a dietary supplement, a symbiotic composition, or a biotherapeutic composition, comprising an effective amount of the composition as defined in any of the claims 1-4, together with appropriate amounts of acceptable excipients or food ingredients.

9. A method to obtain a mutant of the strain of *Lactobacillus casei* deposited in the Spanish Type Culture Collection (CECT) under the accession number CECT 8590, wherein the method comprises using the deposited strain as starting material and applying mutagenesis, and wherein the obtained mutant further retains or enhances at least the ability of the deposited strain to degrade gliadin and the immunotoxic 33-mer peptide having SEQ ID NO: 1.

## Patentansprüche

1. Eine Zusammensetzung umfassend eine wirksame Menge des isolierten Stamms von *Lactobacillus casei*, der bei der spanischen Artenkultursammlung (CECT) unter der Hinterlegungsnummer CECT 8590 hinterlegt ist.

2. Die Zusammensetzung nach Anspruch 1, wobei der isolierte Stamm in einem künstlichen Medium fermentiert worden ist und einer Nachbehandlung nach der Fermentation unterzogen worden ist, um Bakterienzellen zu erhalten, und wobei die entstandenen Bakterienzellen in einem flüssigen Medium oder in fester Form vorhanden sind.

3. Die Zusammensetzung nach Anspruch 2, wobei die Nachbehandlung ausgewählt ist aus der Gruppe bestehend aus: Trocknung, Tiefkühlung, Gefriertrocknung, Fließbetttrocknung, Sprühtrocknung und Kühlung in einem flüssigen Medium.

4. Die Zusammensetzung nach einem der Ansprüche 1-3, wobei der Stamm genetisch verändert worden ist.

5. Die Zusammensetzung wie in einem der Ansprüche 1-4 definiert zur Verwendung als Arzneimittel oder als Probiotikum.

6. Eine Zusammensetzung wie in einem der Ansprüche 1-4 definiert zur Verwendung bei der Prävention und/oder Behandlung von Zöliakie und Störungen, die mit der Aufnahme von Gluten verbunden sind.

7. Eine Zusammensetzung wie in einem der Ansprüche 1-4 definiert zur Verwendung als technologisches Adjuvans.

8. Ein pharmazeutisches Produkt, ein tiermedizinisches Produkt, medizinische Ernährung, ein Nahrungsmittel, ein Lebensmittelergänzungsmittel oder ein Sauerteig, eine probiotische Nahrungsmittelzusammensetzung, ein Nahrungsergänzungsmittel, eine symbiotische Zusammensetzung, oder eine biotherapeutische Zusammensetzung, umfassend eine wirksame Menge der Zusammensetzung wie in einem der Ansprüche 1-4 definiert, zusammen mit geeigneten Mengen von annehmbaren Hilfsstoffen oder Lebensmittelzutaten.

9. Ein Verfahren zum Erhalten von einem Mutanten des Stamms von *Lactobacillus casei*, der bei der spanischen Artenkultursammlung (CECT) unter der Hinterlegungsnummer CECT 8590 hinterlegt ist, wobei das Verfahren folgendes umfasst: das Verwenden des hinterlegten Stamms als Ausgangsmaterial und das Anwenden von Mutagenese, und wobei der erhaltene Mutant weiterhin mindestens die Fähigkeit des hinterlegten Stamms behält oder verbessert, Gliadin und das immunotoxische 33-mer Peptid der SEQ ID NO:1 abzubauen.

## Revendications

1. Une composition comprenant une quantité efficace de la souche isolée de *Lactobacillus casei* déposée dans la collection espagnole de cultures type (CECT) sous le numéro de dépôt CECT 8590.

2. La composition selon la revendication 1, dans laquelle la souche isolée a été fermentée dans un milieu artificiel et soumise à un traitement ultérieur après la fermentation pour obtenir des cellules bactériennes, et dans laquelle les cellules bactériennes résultantes sont dans un milieu liquide ou sous forme solide.

3. La composition selon la revendication 2, dans laquelle le traitement ultérieur est choisi dans le groupe constitué de : séchage, congélation, lyophilisation, séchage à lit fluidisé, séchage par atomisation et réfrigération dans un milieu liquide.

4. La composition selon l'une quelconque des revendications 1-3, dans laquelle la souche a été génétiquement modifiée.

5. La composition telle que définie dans l'une quelconque des revendications 1-4, pour l'utilisation comme médicament ou comme probiotique.

6. Une composition telle que définie dans l'une quelconque des revendications 1-4 pour l'utilisation dans la prévention et/ou le traitement de la maladie coeliaque et les troubles liés à l'apport de gluten.

7. Une composition telle que définie dans l'une quelconque des revendications 1-4, pour l'utilisation comme adjuvant technologique.

8. Un produit pharmaceutique, un produit vétérinaire, un aliment médical, un produit alimentaire, un supplément alimentaire ou un levain panaire, une composition alimentaire probiotique, un supplément nutritionnel, une composition symbiotique, ou une composition biothérapeutique, comprenant une quantité efficace de la composition telle que définie dans l'une quelconque des revendications 1-4 conjointement avec des quantités appropriées d'excipients ou d'ingrédients alimentaires acceptables.

9. Un procédé pour obtenir un mutant de la souche de *Lactobacillus casei* déposée dans la collection espagnole de cultures type (CECT) sous le numéro de dépôt CECT 8590, dans lequel le procédé comprend utiliser la souche déposée comme produit de départ et appliquer de la mutagénèse, et dans lequel le mutant obtenu retient ou améliore en outre au moins la capacité de la souche déposée de dégrader la gliadine et le peptide 33-mer immunotoxique ayant la SEQ ID No : 1.


### SEQUENCE LISTING

<110> CONSEJO SUPERIOR DE INVESTIGACIONES CIENTÍFICAS (CSIC)
<120> NEW STRAIN OF Lactobacillus casei WITH ABILITY TO DEGRADE THE IMMUNOTOXIC PEPTIDE FROM GLUTEN
<130> PD10109PC00
<150> ES201430768
   <151> 2014-05-23
<160> 1
<170> PatentIn version 3.5
<210> 1
   <211> 32
   <212> PRT
   <213> Triticum aestivum
<400> 1
   Leu Gln Leu Gln Pro Phe Pro Gln Pro Gln Leu Pro Tyr Pro Gln Pro
   1               5                   10                  15
   Leu Pro Tyr Pro Gln Pro Gln Leu Pro Tyr Pro Gln Pro Gln Pro Phe
               20                  25                  30